# EUROPEAN PATENT APPLICATION

(11) **EP 2 975 137 A1**
(43) Date of publication of application: **20.01.2016**
(21) Application number: 14306170.3
(22) Date of filing: 18.07.2014
(51) Int. Cl.: C12Q 1/68

(54) **Use of immune diversity as a predictive marker for identifying B-cell-lymphomas-patients having an increased risk of febrile or infectious event**

(71) Applicant: ImmunID, 38040 Grenoble (FR)
(72) Inventor: Filipe Santos, Orchidée, 38520 LE BOURG D'OISANS (FR); Pasqual, Nicolas, 38500 COUBLEVIE (FR); Courtier, Anaïs, 38500 VOIRON (FR); Weisbuch, Sébastien, 38430 SAINT JEAN DE MOIRANS (FR); Mouret, Jean-François, 38500 COUBLEVIE (FR)
(74) Representative: Marcadé, Véronique

(57) **Abstract**

The present invention pertains to the field of cancer therapy and provides a predictive marker for determining if a patient suffering from a B-cell neoplasm can benefit from an anti-CD20-based chemotherapy.

## Description

The present invention pertains to the field of anti-cancer medicine. More precisely, the present invention provides a new tool for assessing the risk of occurrence of a febrile and/or infectious event for a patient having a B-cell neoplasm, and deducing the need, for this patient, of an antibacterial and/or antifungal prophylactic treatment. The invention also provides a method of evaluating the risk-benefit balance of administering an anti-CD20-based chemotherapy to a patient having a B-cell neoplasm.

Lymphoid neoplasms include tumours originating in lymphoid tissue that comprise lymphoid leukaemia such as acute (ALL) and chronic leukaemia (CLL), as well as a diverse yet closely related group of hemopathies, including non-Hodgkin lymphoma (NHL), Hodgkin lymphoma (HL) and multiple myeloma. Together, lymphoid neoplasms yielded approximately 93,420 incident cases and 38,000 deaths in the United States in 2005. According to World Health Organization (WHO) data and classification, NHL are the most frequent lymphoid neoplasms (35%), followed by multiple myeloma (15%) and CLL (12%) (Morton et al., 2006), Amongst NHLs, diffuse large B cell lymphoma (DLBCL) is the most common, accounting for 30-40% of NHLs worldwide (Gascoyne, 2014, Siegel et al., 2013), followed by follicular lymphoma (FL, 22%) and marginal zone lymphoma (MALT, 7%) (Morton et al. 2006).

Most of lymphoid neoplasms were usually considered as incurable, but patients' outcome has been steadily improving over the last decade. The introduction of anti-CD20 monoclonal antibodies represented a major improvement in overall survival of these patients (Récher et al., 2011). Rituximab (trade names Rituxan®, MabThera® and Zytux® from Genentech, Hoffmann-La Roche), which is the most commonly used, is a chimeric monoclonal antibody against the protein CD20, a molecule that is primarily found on the surface of immune system B cells. Rituximab destroys B cells and is therefore used to treat diseases which are characterized by an excessive number of B cells, overactive B cells, or dysfunctional B cells. Rituximab was approved by the FDA in 1997 for the treatment of NHL and CLL in combination with chemotherapy (Czuczman et al. 1999). According to Rituximab's medication guide, the use of this anti-CD20 antibody is also indicated in the treatment of several diseases other than haematological cancers, such as rheumatoid arthritis (RA), Granulomatosis with Polyangiitis (GPA) (Wegener's Granulomatosis) and Microscopic Polyangiitis (MPA). In addition, Rituximab has been shown to be effective in several other autoimmune diseases such as multiple sclerosis (MS), systemic lupus erythematosus (SLE) and autoimmune anaemia, autoimmune hemolytic anemia, pure red cell aplasia, idiopathic thrombocytopenic purpura (ITP), Evans syndrome, vasculitis, bullous skin disorders, type 1 diabetes mellitus, Sjogren's syndrome, Devic's disease and Graves' ophthalmopathy. Rituximab is also being used off-label in the management of organ transplant recipients such as kidney transplant.

Since the FDA approval of Rituximab in 1997, the efficacy of this anti-CD20 antibody has been worldwide accepted and its success has led to the developments of some other anti-CD20 monoclonal antibodies, such as conjugated and/or humanized anti-CD20 antibodies: I131-tositumomab (trade name Bexxar®), a murine IgG2 anti-CD20 monoclonal antibody covalently bound to the radionuclide iodine-131; Obinutuzumab (called afutuzumab until 2009; approved under the trade name Gazyva® by the US FDA in 2013), a humanized monoclonal anti-CD20 antibody; Y90-ibritumomab tiuxetan (trade name Zevalin®), a murine monoclonal antibody that is bound to the radioactive isotope chelator tiuxetan for radioimmunotherapy; ocretizumab, a humanized (90%-95% human) B cell-depleting agent (Genentech, Hoffmann-La Roche); ofatumumab (HuMax-CD20), a fully human unconjugated anti-CD20 antibody (trade name Arzerra®, from Genmab); and more recently, third-generation anti-CD20s were developed, with a glycoengineered Fc fragment (Fc) having enhanced binding to Fc gamma receptors, which increases ADCC (antibody-dependent cellular cytotoxicity). However, no clear added value of a humanized molecule, compared to the original design, has been demonstrated so far in oncology.

In addition to anti-CD20 antibodies, a number of other molecules have been used as targets to develop antibodies such as anti-CD52, anti-CD30 and anti-CD19. Alemtuzumab, which is a humanized anti-CD52 monoclonal antibody used primarily for CLL, seems to be more effective for induction and maintenance therapy. Alemtuzumab is typically not combined with chemotherapy due to increased risk of infection as compared with anti-CD20. Brentuximab vedotin ADCETRIS (Seattle Genetics, Inc., Bothell, WA, USA) comprises the chimeric CD30 targeted monoclonal antibody, which is linked to three to five units of the antimitotic agent monomethyl auristatin E. Brentuximab vedotin was approved for treatment of anaplastic large cell lymphoma and Hodgkin lymphoma. SAR3419 (huB4-DM4) is a novel antibody-drug conjugates that is composed of a humanized monoclonal IgG1 anti-CD19 antibody (huB4) attached to the potent cytotoxic drug, a maytansine derivative an antimitotic agent. Preclinical efficacy of maytansine derivative-anti-CD 19 conjugate was demonstrated and phase I trials have also demonstrated promising antitumor activity with acceptable safety results in human B-cell lymphoma models. Additional trials are ongoing and will provide additional insight into the full potential of this novel drug (Al-Katib et al., 2013).

Diffuse large B cell lymphoma (DLBCL) is an aggressive B-cell NHL that affects patients of all ages with a wide range of clinical presentations. Patients with DLBCL show marked morphologic, phenotypic, genetic and clinical heterogeneity. The current standard for care, especially in elderly patients, is immunochemotherapy composed of rituximab, doxorubicin, cyclophosphamide, vincristine, and prednisone (abbreviated R-CHOP) that produces cures in ∼65-70% of patients. In some patients especially youngers, aged 18-59 years, a significant improved survival can be achieved with intensified immunochemotherapy such as rituximab, doxorubicin, cyclophosphamide, vindesine, bleomycin, and prednisone (abbreviated R-ACVBP), but also with significant increase of haematological toxic effects (Recher et al., 2011). Therefore, R-CHOP is the standard for care of DLBCL affected patients of all ages; but R-ACVBP may be a therapeutic option in younger. DLBCL is now curable, even in advanced stages; however, still up to one-third of patients do not achieve cure with initial therapy and the prognosis for these patients is poor (Roschewski et al., 2014).

Follicular B cell lymphoma (FL) is the second most frequent NHL subtype, accounting for about 10%-20% of all lymphomas in western countries (Ghielmini et al., 2013; Morton et al., 2006). FL is characterized by a marked clinical heterogeneity; some patients are symptomatic for many years, while others rapidly present a life-threatening disease. The tumor burden criteria and the follicular lymphoma international prognostic index (FLIPI) may help to stratify patients for treatment decisions. For patients affected by FL with a high tumor burden or who develop symptoms after a watchful waiting period, the use of cytotoxic chemotherapy combined with rituximab such as R-CHOP has been established as the standard for care in the last decade. Like DLBCL, the introduction of anti-CD20 monoclonal antibodies improved survival of FL patients and recent epidemiological projection showed that more than 70 % of the FL patients over the age of 60 may survive for at least 10 years, (Salles and Ghesquières, 2012).

Chronic lymphocytic leukaemia (CLL) is the most common form of adult leukaemia in western countries; it accounts for approximately 30-40% of all leukaemias and remains incurable with conventional chemotherapy treatment approaches. CLL typically occurs in elderly patients and has a highly variable clinical course. Leukemia transformation is initiated by specific genomic alterations that impair apoptosis of clonal B-cells. Two prognostic staging systems exist, the Rai and Binet staging systems, which are established by physical examination and blood counts. Various biological and genetic markers also have prognostic value: deletion of the short arm of chromosome 17 (del(17p)) predicts resistance to most available therapies (Hallek, 2013); the immunoglobulin heavy chain variable gene (IGHV) mutational status has been recognized as an important predictor of prognosis in chronic lymphocytic leukaemia (CLL), as well as, more recently, other features of the B-cell receptor and different genes belonging to the IGHV3 subgroup (Dal-Bo et al., 2011). In addition, some studies have shown that survival of CLL cells strictly depends on a permissive microenvironment composed of cellular components like macrophages, T cells, or stromal follicular dendritic cells (Burger et al., 2009). Only patients with active or symptomatic disease or with advanced Binet or Rai stages require therapy. There is an impressive choice of combination options for the treatment of active CLL, and the management of CLL is currently undergoing a dynamic and rapid change. However, here again, rituximab combined with chemotherapy such as fludarabine/cyclophosphamide (abbreviated RFC) proved to be very a efficacious therapy for CLL (Hallek, 2013). The substantial clinical variability in the clinical course of CLL and treatment options, has motivated intense efforts at identifying molecular markers that can be used for CLL prognostication (Malek, 2013) and that can help clinicians to choose the right treatment for a given CLL patient.

A common side-effect of all these chemotherapy treatments is the hematologic toxicity. These treatments are considered to be myelotoxic, affecting bone marrow and resulting in impaired blood cell count. A large proportion of treated patients experienced serious adverse event, notably febrile episodes or infections. Infection is one of the most frequent haematological toxic effects of the intensive chemotherapy regimen and for some 10 to 20% of infection episodes, hospitalization is required (Cullen et al., 2005; Morrison, 2010). This phenomenon is not exclusive to the treatment of patients affected by NHL or leukaemia, but it has been reported in solid cancers such as testicular, small-cell lung and breast cancer (Cullen et al., 2007), for which the anticancer treatments are also known to be myelotoxic. The progress in treatment strategies, leading to is more aggressive and efficacious supportive care, has recently improved the prognosis of patients with malignancies, but these advances have also increased the risk of severe infections due to a more profound level of immunosuppression (neutropenia, in particular) and/or the extensive use of medical devices (Trecarichi and Tumbarello, 2014). In the treatment of haematological cancers, this phenomenon is increased by the introduction of anti-CD20-based therapies. Serious infections can happen during and after treatment with anti-CD20, and can lead to death. Various types of serious bacterial, fungal, and viral new infections or reactivated viral infections can occur during and following the completion of anti-CD20-based chemotherapy.

Infections are the leading cause of premature discontinuation and non-relapse mortality. Broad-spectrum antibiotic therapy is introduced as soon as an infectious problem is suspected (Cabanillas et al., 2006). The role of antibiotic prophylaxis in patients who received myelotoxic chemotherapy remains controversial and few studies on this topic are found in scientific and medical literature. Some studies have shown that antibacterial prophylaxis led to a reduction of febrile events and documented infections, their tolerability is generally good and hospitalization may be avoided or shortened, resulting in cost savings (Neumann et al., 2013). Some others have shown that treatment with antibacterial prophylaxis concomitant to chemotherapy has significantly reduced the occurrence of febrile episodes and infections, leading to a decrease of hospitalizations of patients with severe grade 4 neutropenia (Cullen et al., 2005). Additional studies from these data showed that the effect of antibiotic treatment appears to be more effective when administered after the first cycle of chemotherapy (Cullen et al., 2007). The concept of antibiotic prophylaxis is however still challenged because of possible side effects such as development of resistance and toxicity of the drugs (Trecarichi and Tumbarello, 2014). Therefore, only patients at high risk of serious infections should be considered for antibiotic prophylaxis.

Neutropenia was described as a blood indicator monitored in the onco-haematological context (Tadmor et al., 2013). However, studies showed that even patients with normal neutrophil counts experienced febrile episodes and infections (Cabanillas et al., 2006; Tadmor et al., 2013).

In this context, finding prognostic factors for the occurrence of febrile episodes and infections is an important issue, even for patients with no neutropenia or with low grade (1 or 2) neutropenia, in order to rationally select patients who may benefit from antibiotic prophylaxis. Besides, there is an important need for methods, tools or markers to help the physicians in their decisions to administer very aggressive treatments such as anti-CD20-based chemotherapy to cancer patients. Indeed, although these treatments can cure cancers which were beforehand considered as incurable, they can also provoke serious or even lethal side-effects, and the clinicians need tools to evaluate the benefit-risk balance of such a treatment, for an individual patient.

In the experiments reported below, the inventors analyzed the T cell repertoire of patients having a B-cell neoplasm, to find prognostic factors for the occurrence of infections. Indeed, recent results support the hypothesis that rituximab interferes with T cell functioning (Avivi et al., 2013). The results reported below demonstrate that T cell immune repertoire profiling can be used as a predictive tool of adverse events during the treatment of haematological cancers, notably the occurrence of early infections during treatment.

A first aspect of the present invention is hence an *in vitro* method of predicting the occurrence of a febrile and/or infectious event in a patient suffering from a B-cell neoplasm, comprising: measuring diversity of the immune repertoire in a biological sample from said patient and comparing the measured diversity to a predetermined threshold, wherein a measured diversity lower than said threshold is indicative of an increased risk of occurrence of a febrile and/or infectious event, and a measured diversity higher than said threshold is indicative of a decreased risk of occurrence of a febrile and/or infectious event.

According to a particular embodiment of the above method, the patient received or will receive a treatment comprising an anti-CD20 molecule (hereafter called "an anti-CD20-based treatment/chemotherapy").

This method is particularly useful for *in vitro* determining if a patient suffering from a B-cell neoplasm needs an antibacterial and/or antifungal and/or another prophylactic treatment such as healthy life style recommendations (hygiene procedures, cooked food, avoid non cooked food, eat special nutriments...) or therapeutic intervention. Indeed, a measured diversity lower than a predetermined threshold is indicative of a need, for the patient, of such a prophylactic treatment, and a measured diversity higher than said threshold indicates that the patient does *a priori* not need such a prophylactic treatment or other preventive treatments.

The present invention also relates to an *in vitro* method of obtaining data to assess the risk-benefit balance of an anti-CD20-based chemotherapy for a patient suffering from a B-cell neoplasm, comprising a step of measuring diversity of the immune repertoire in a biological sample from said patient and a step of comparing this diversity to at least one predetermined threshold, wherein the potential benefits of the anti-CD20-based chemotherapy outweigh its risks for the patient if the measured diversity is above said predetermined threshold. According to a particular embodiment, this method can be performed with two thresholds: if the patient's diversity is above the upper threshold, the anti-CD20-based chemotherapy is administered to the patient without any prophylactic (antibacterial, antifungal *etc*.) treatment, whereas it is administered together with such a prophylactic treatment if the patient's diversity is between the two predetermined thresholds, and the risk is considered as too important for this patient to receive an anti-CD20 molecule if the patient's diversity is below the lower threshold.

Another aspect of the present invention is hence a method for taking care of a patient suffering from a B-cell neoplasm, comprising a step of measuring diversity of the immune repertoire in a biological sample from said patient and a step of administering an anti-CD20-based chemotherapy, alone or with prophylactic complements to said patient, depending on the measured diversity (compared to one or several predetermined thresholds).

As defined above, an "anti-CD20-based chemotherapy" is a treatment comprising administering an anti-CD20 molecule. According to a preferred embodiment, the anti-CD20 chemotherapy comprises administering an anti-CD-20 monoclonal antibody. Non-limitative examples of anti-CD20 antibodies are recalled above. Particular examples of anti-CD20 chemotherapies which can be more appropriately given to patients suffering from B-cell neoplasm are R-CHOP (immunochemotherapy composed of rituximab, doxorubicin, cyclophosphamide, vincristine, and prednisone) and R-ACVBP (combination of rituximab, doxorubicin, cyclophosphamide, vindesine, bleomycin, and prednisone).

In a prospective study reported below, the inventors performed a dynamic analysis of TRB and IGH repertoires over time, in patients having a FL or a DLBCL and treated with an anti-CD20-based chemotherapy. By doing so, they showed that results obtained shortly after cycle 1 of the treatment were even more significant than those obtained at diagnosis. Hence, according to a preferred embodiment of the above methods, the biological sample has been collected less than 18 to 24 days before or after the second administration of the treatment, even more preferably less than 7 days before or after the second administration of the treatment.

According to a preferred embodiment of the above methods the patient suffers from a B lymphoma, for example from a large B cell lymphoma (DLBCL) or a follicular lymphoma (FL).

More generally, the present invention pertains to an *in vitro* method of obtaining information relating to the ability of a patient suffering from a cancer to benefit from an aggressive treatment and/or information to assess the risk-benefit balance of such a treatment for said patient, comprising a step of measuring diversity of the immune repertoire in a biological sample from the patient and a step of comparing this diversity to at least one predetermined threshold, wherein the patient is likely to support the aggressive treatment if the measured diversity is above said predetermined threshold. The treatment's potential benefits then outweigh its risks for the patient, and the aggressive treatment can be administered. This method can in particular be applied to patients suffering from a B-cell neoplasm, to assess the risk-benefit balance of therapies with anti-CD52, anti-CD30 or anti-CD19 antibodies for these patients.

The above methods can be performed from any biological sample enabling the determination of the diversity of the immune repertoire. Non-limitative examples of such samples include a whole blood sample, a blood clot, PBMCs, a tissue biopsy, *etc.*

According to a preferred embodiment of the present invention, TCR diversity is measured. In particular, the measured TCR diversity can include or consist of TCR combinatorial diversity. According to a particular way of conducting the methods according to the invention, illustrated in the experimental part below, the diversity of the immune repertoire is measured by a multiplex PCR assay allowing the simultaneous detection of a significant number of TRBV-TRBJ rearrangements. For example, this assay can be designed so as to detect the presence or absence (and/or the level) of at least 20, preferably at least 35, at least 50, at least 100 or at least 200 TRBV-TRBJ rearrangements. Of note, the results shown in the experimental part below have been obtained by analyzing 276 different TRBV-TRBJ rearrangements covering 100% of the possible combinatorial rearrangements, but the skilled artisan can select, among these, the most informative ones, and/or use primers allowing the amplification of rearrangement representative of mono- or multi-gene families, in order to routinely perform the above-described method with a decreased complexity, for example by amplifying fragments from no more than 20 TRBV-TRBJ rearrangements.

It is important to note that in the frame of the present invention, several parameters of the immune diversity can be measured. In particular, the diversity can be expressed as a percentage of the number of rearrangements which can theoretically be observed by the technology used to measure it (called "global diversity" or "diversity richness" in the following text). For example, when the measured diversity is the combinatorial diversity and is measured by a PCR assay allowing the detection of 150 TRBV-TRBJ rearrangements if they are present in the sample, the global diversity of a sample in which 75 rearrangements are observed is 50%. Another parameter of the immune diversity can advantageously be measured when performing the method according to the present invention is the percentage of observed rearrangements which are responsible for a certain amount (X%) of contribution amongst the observed rearrangements. This parameter, called "divX", reflects the evenness of the rearrangements present in the sample. For example, div50 corresponds to the percentage of rearrangements necessary to reach 50% of contribution amongst observed rearrangements. DivX (wherein X is comprised between 0 and 100, preferably between 20 and 80) is a parameter of diversity allowing to characterize the level of evenness of an immune repertoire by focusing on the most important rearrangements in term of contribution to the whole repertoire. The lower the value of divX, the more restricted the repertoire.

Of course, the skilled artisan will adapt the predetermined threshold(s) to the parameter which is measured and to the technology used to measure it by assessing, in a given cohort and with this technology, which couple (X, threshold) provides the best sensitivity and specificity (ROC curves can be used to this aim). Generally, the threshold to be considered when performing the above methods is predetermined by measuring the diversity of the immune repertoire in a representative cohort of individuals (for example, patients having a B-cell neoplasm and who received an anti-CD20-base chemotherapy), whose capacity to support a given aggressive treatment is known. The threshold is calculated to obtain the best predictability (sensitivity and specificity) for the response / patient's outcome / occurrence of adverse events.

For example, as disclosed in Example 3 below, when the combinatorial diversity of the immune repertoire of a DLBCL patient is measured shortly after the first cycle of treatment with an anti-CD20-based chemotherapy, in a blood clot, with a technology similar to the "protocol A" used in the experimental part and expressed as a percentage of observed rearrangements, a threshold of 56%, gives good results for predicting the occurrence of a febrile or infectious event. For the same cohort, but if the diversity is measured with a more potent protocol ("protocol B"), a predetermined threshold of around 80%, can be considered.

According to a particular embodiment of a method as above described, a patient suffering from a B lymphoma and having a combinatorial TRB diversity ≤ 58% 18 to 24 days after the first administration of an anti-CD20-based treatment is identified at risk of infection and as being in need of an antibacterial and/or antifungal and/or other prophylactic treatment (such as healthy life style recommendations or other preventive treatments) or therapeutic intervention.

According to another particular embodiment of a method as above described, a patient suffering from DLBCL and having a combinatorial TRB diversity ≤ 58% 18 to 24 days after the first administration of a treatment comprising an anti-CD20 molecule is identified as being in need of an antibacterial and/or antifungal prophylactic treatment, healthy life style recommendations or other preventive treatments.

According to yet another particular embodiment of a method as above described, a patient suffering from FL and having a combinatorial TRB diversity ≤ 64% 18 to 24 days after the first administration of a treatment comprising an anti-CD20 molecule is identified as being in need of an antibacterial and/or antifungal prophylactic treatment, healthy life style recommendations or other preventive treatments.

Of course, the skilled artisan is free to re-evaluate each of these thresholds on a larger cohort of patients, and by using any kind of technology for measuring the diversity of the immune repertoire (such as, for example, Next Generation Sequencing (NGS), spectratyping, immunoscope, Flow cytometry analysis...). The skilled artisan can also refine the threshold for particular subpopulations, depending on the type of cancer, the specific drug considered for the treatment, or any other parameter regarding the patients, their pathologies (age of patient, associated treatment, type of comorbidities, ...) or their treatment (coadministration of another antineoplastic drug, different dosage regimen, *etc*.). Of course, the threshold also depends upon the parameter(s) used to assess the diversity of the immune repertoire (global diversity, Div25, Div50, *etc*.) and the skilled artisan can perfectly determine an optimal couple (parameter, threshold) or an optimal triplet (technology, parameter, threshold) to obtain a predictive method as sensitive and specific as possible.

The present invention will be understood more clearly from the further description which follows, which refers to examples illustrating the predictive value of the combinatorial TRB diversity, to assess the risk of infection for a patient suffering from a B-cell lymphoma, as well as to the appended figures.

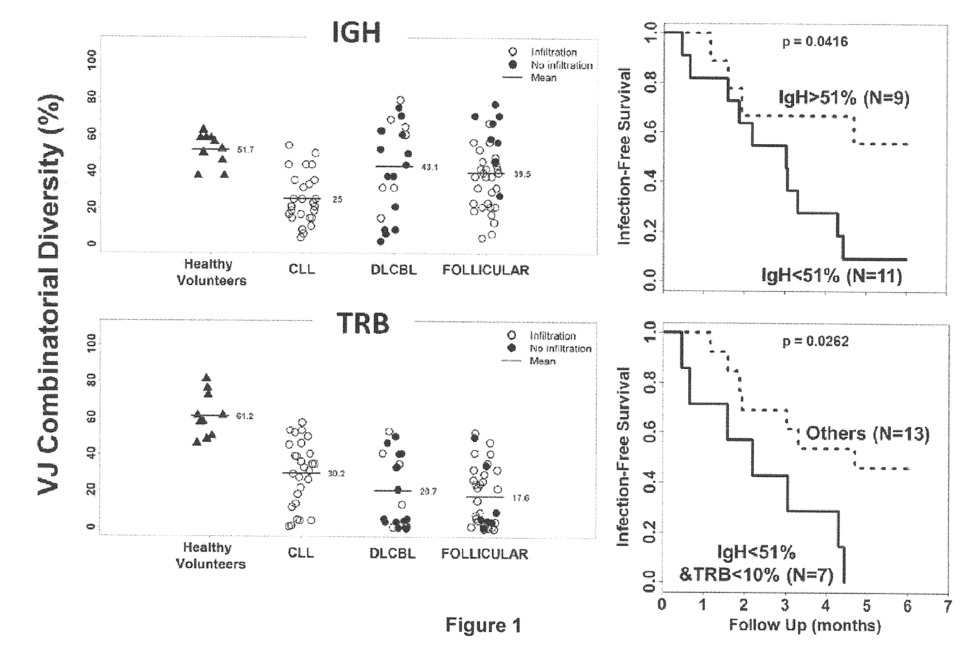

**Figure 1****:** RIPAL retrospective study. Analysis of IGH and TRB diversity at diagnosis in chronic leukemia and NHL such as DLBCL and FL (left panels). At diagnosis, IGH diversity alone or in combination with TRB diversity is predictive for the occurrence of first infection by the end of M6 in DLBCL patients treated with Rituximab plus chemotherapy. Optimal IGH and TRB threshold were obtained through ROC curves analysis.

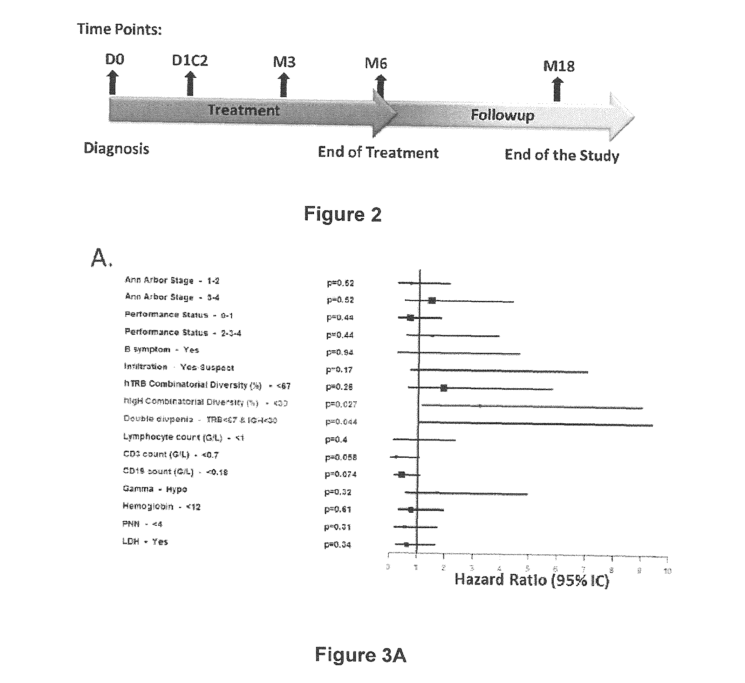

**Figure 2****:** RIPAL prospective study design. Blood samples, clinical and biological data were collected at diagnosis (D0); first day of second cure cycle (D1C2); and then 3 (M3); 6 (M6) and 18 months (M18) after treatment. All patients were followed 18 months after diagnosis date. M6 is the theoretical date of the end of treatment, although some patients were still treated after this period.

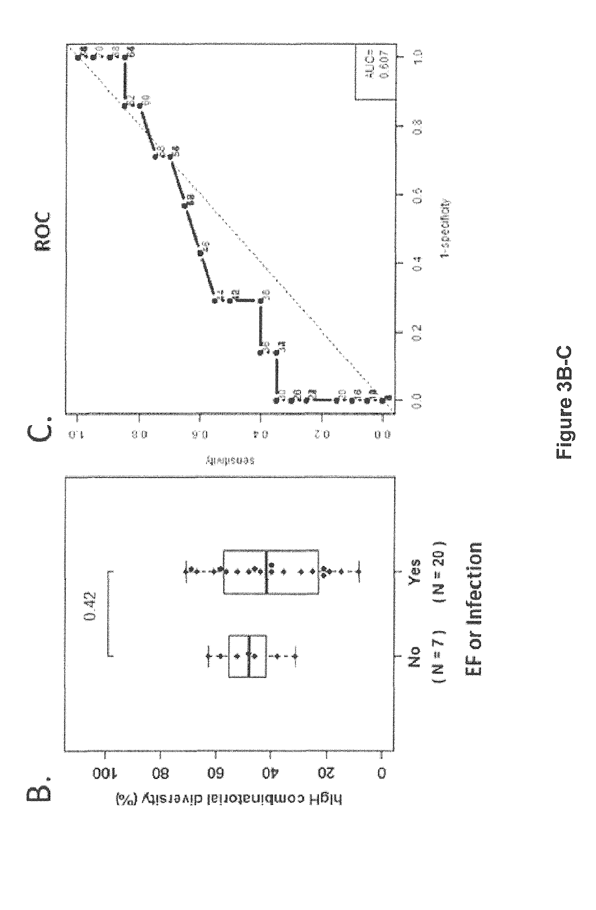

**Figure 3****:** RIPAL prospective study. Analysis of prognostic factors at baseline (DO) of the febrile episode and/or infection occurrence by the end of M6 in DLBCL patient. A. The forest plot shows hazard ratios according to characteristics of the patients, univariate Cox model analysis highlights a small but significant risk of IGH diversity on the threshold of 30%, B. despite that no significant difference in levels of diversity IGH were found between patients who developed (yes) or not (no) infection by the end of M6 posttreatment. C. ROC analysis showed a weak discrimination performance (AUC = 0.607) of IGH diversity levels on the occurrence of a febrile episode and/or infection.

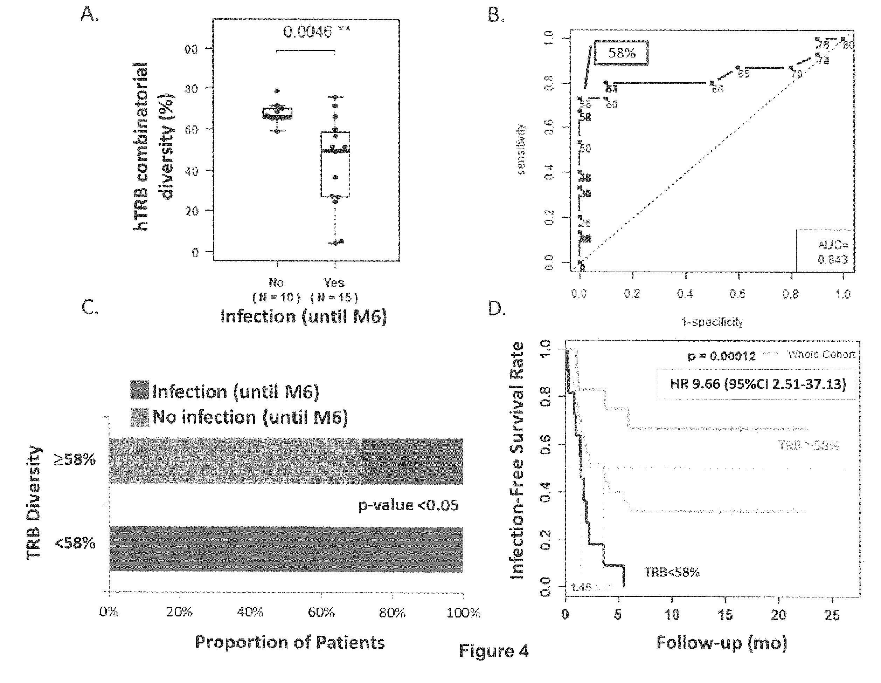

**Figure 4****:** RIPAL prospective study. Analysis of TRB diversity as predictive factor of the occurrence of first infection by the end of M6 in DLBCL patients treated with Rituximab plus chemotherapy. A. Comparison of TRB diversity levels at day 21 after treatment (D1C2) between patients who developed (yes) or not (no) at least one infection by the end of M6 shows a significant difference between the two (Wilcoxon p <0.05). B. Comparison of TRB diversity levels between patients who developed (yes) or not (no) febrile episodes or infection shows a significant difference between the two (Wilcoxon p <0.005). C. ROC analysis showed a strong discrimination performance for TRB diversity at D1C2 (AUC = 0.843) an optimal threshold at 58%. D. Kaplan-Meier estimates of infection-free survival curve of DLBCL patients stratified according to Youden's index at 58% TRB diversity lower (red) or higher (green). The median duration of infection-free survival was 1.45 months in the group TRB<58% as compared with the group TRB >58% who didn't reach median duration over the follow-up period, corresponding to a significant (log rank p <0.001) hazard ratio (HR) for progression of 9.66 (95%CI 2.51-37.13).

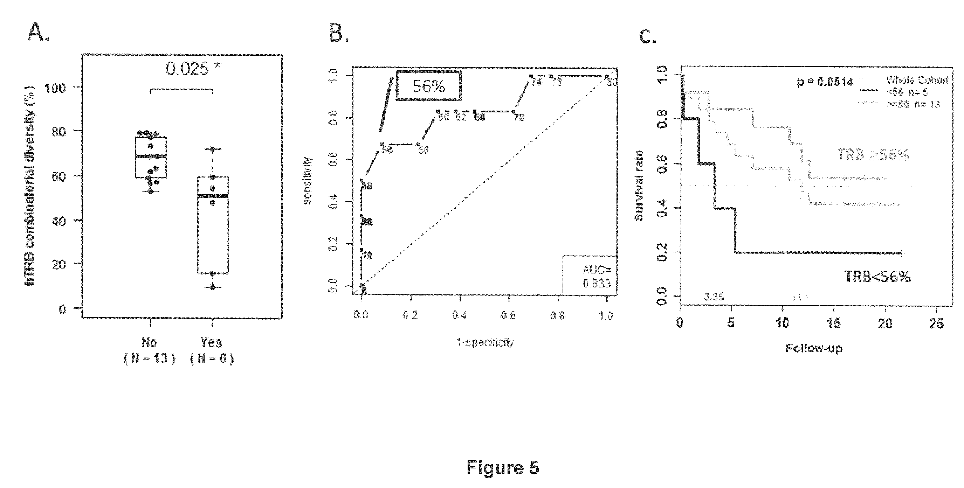

**Figure 5****:** RIPAL prospective study. Analysis of TRB diversity as predictive factor of the occurrence of first infection by the end of M6 in FL patients treated with Rituximab plus chemotherapy. A. Comparison of TRB diversity levels at D0 (before treatment) between patients who developed (yes) or not (no) at least one infection by the end of M6 shows a difference but not significant between the two (Wilcoxon p >0.05). B. ROC analysis showed a strong discrimination performance for TRB diversity at DO (AUC = 0.833) an optimal threshold at 56%. C. Kaplan-Meier estimates of infection-free survival curve of FL patients stratified according to Youden's index at 56% TRB diversity. The median duration of infection-free survival was 3.35 months in the group TRB<56% as compared with the group TRB ≥56% who didn't reach median duration over the follow-up period, but the difference is on the limit of significance (log rank p ∼0.05).

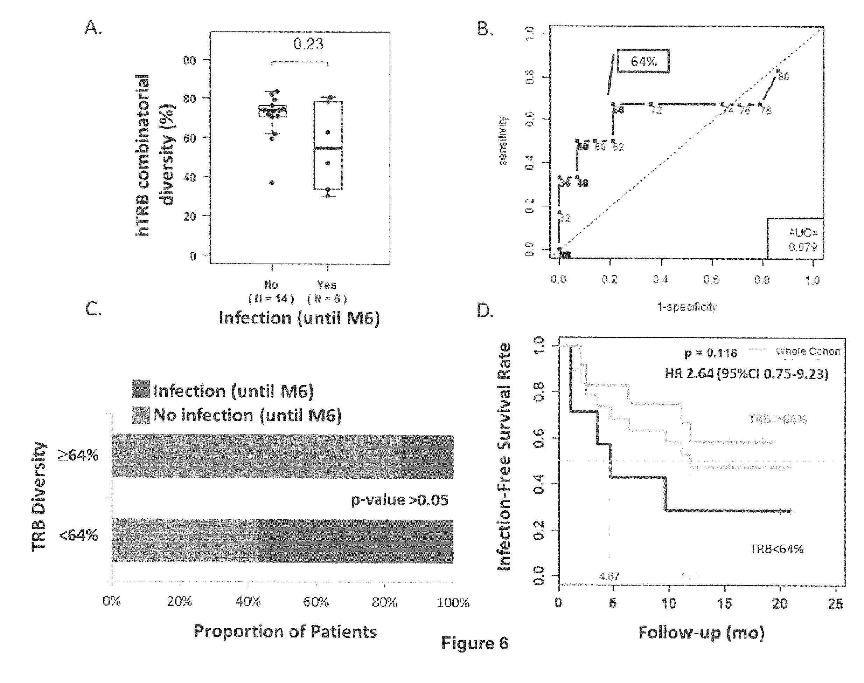

**Figure 6****:** RIPAL prospective study. Analysis of TRB diversity as predictive factor of the occurrence of first infection by the end of M6 in FL patients treated with Rituximab plus chemotherapy. A. Comparison of TRB diversity levels at day 21 after treatment (D1C2) between patients who developed (yes) or not (no) at least one infection by the end of M6 shows a difference but not significant between the two (Wilcoxon p >0.05). B. ROC analysis showed a strong discrimination performance for TRB diversity at D1C2 (AUC = 0.679) with an optimal threshold at 64%. C. The proportion of patients who experienced an infection until M6 is higher in those whose TRB diversity <64%, the difference is not significant (Fischer's test p>0.05). D. Kaplan-Meier estimates of infection-free survival curve of FL patients stratified according to Youden's index at 64% TRB diversity. The median duration of infection-free survival was 4.67 days in the group TRB<64% as compared with the group TRB >64% who did not reach median duration over the follow-up period, corresponding to a hazard ratio (HR) for progression of 2.64 (95%CI 0.75-9.23) the difference was not significant (log rank p >0.05).

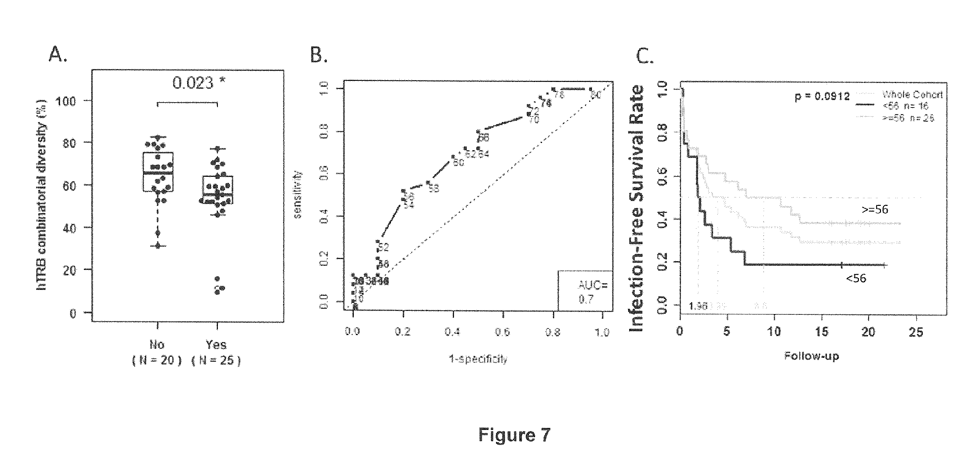

**Figure 7****:** RIPAL prospective study. Analysis of TRB diversity as predictive factor of the occurrence of first infection by the end of M6 in FL plus DLBCL patients treated with Rituximab plus chemotherapy. A. Comparison of TRB diversity levels at D0 between patients who developed (yes) or not (no) at least one infection by the end of M6 shows a significant difference between the two (Wilcoxon p >0.05). B. ROC analysis showed a discrimination performance for TRB diversity at D0 (AUC = 0.7) with an optimal threshold at 56%. C. Kaplan-Meier estimates of infection-free survival curve of FL patients stratified according to Youden's index at 56% TRB diversity. The median duration of infection-free survival was 1.96 months in the group TRB<56 % as compared 8.81 months with the group TRB >56%, but the difference is on the limit of significance (log rank p >0.05).

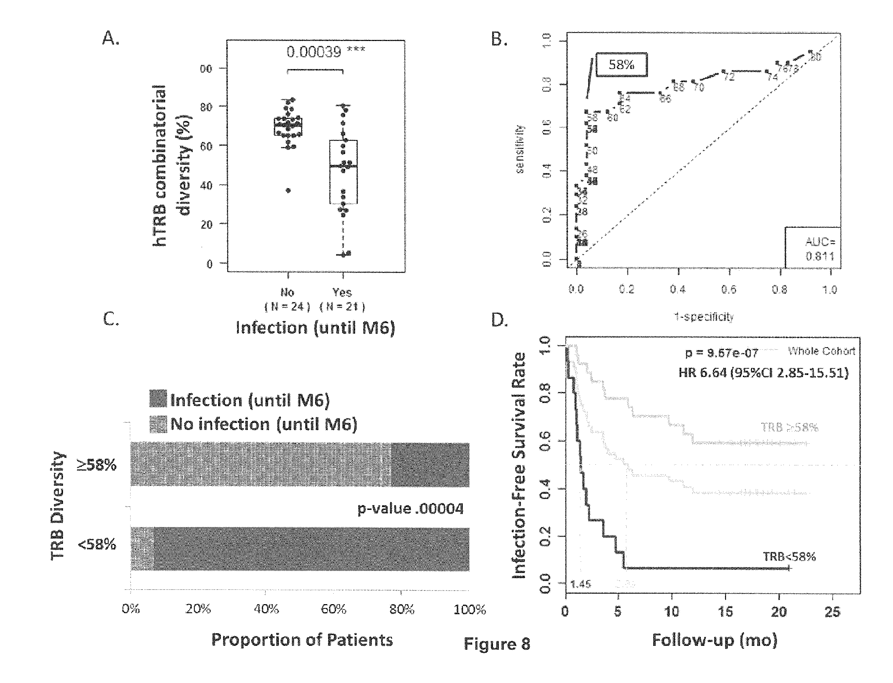

**Figure 8****:** RIPAL prospective study. Analysis of TRB diversity as predictive factor of the occurrence of first infection by the end of M6 in pooled DLBC plus FL patients treated with Rituximab-combined chemotherapy. A. Comparison of TRB diversity levels at day 21 after treatment (D1C2) between patients who developed (yes) or not (no) at least one infection by the end of M6 shows a significant difference between the two (Wilcoxon p 0.00034). B. ROC analysis showed a strong discrimination performance for TRB diversity at D1C2 (AUC = 0.811) with an optimal threshold at 58%. C. The proportion of patients who experienced an infection until M6 is higher in those whose TRB diversity <58%, the difference is significant (Yates'Chi2 p 0.0004). D. Kaplan-Meier estimates of infection-free survival curve of pooled patients stratified according to Youden's index at 58% TRB diversity. The median duration of infection-free survival was 1.45 days in the group Try<58% as compared with the group TRB ≥58% who did not reach median duration over the follow-up period, corresponding to a hazard ratio (HR) for progression of 6.64 (95%CI 2.85-15,51); the difference is strongly significant (log rank p <<0.05).

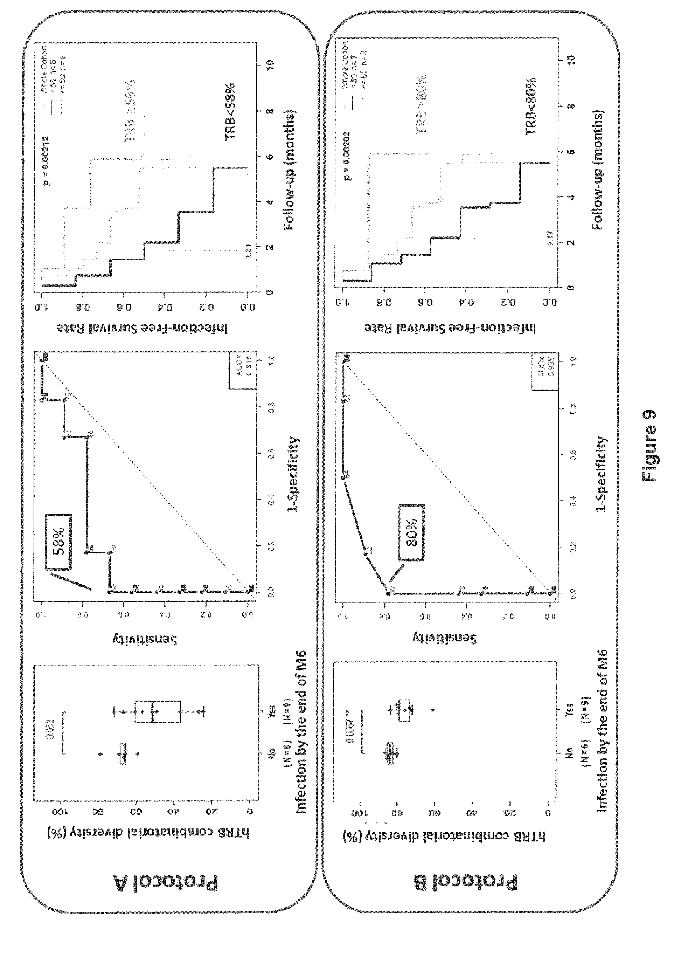

**Figure 9****:** RIPAL prospective study. Comparison of TRB results obtained through different protocols. Fifteen DLBCL patients' samples were randomly selected in order to compare clinical validity of TRB diversity evaluated through different technical protocols. Protocol A analytically validated for RIPAL study was applied for the analysis of the entire study. Protocol B is an improved version of protocol A. Left panels: comparisons of TRB diversity levels between DLBCL patients who developed (yes) or not (no) an infection by the end of M6. Middle panels: receiver operating characteristic (ROC) curves, to assess discrimination capacity of TRB levels to infection outcome; optimal cut-off point of discrimination is obtained though Youden index calculation, which is represented in the box. Right panels: Youden's index was used to stratify patients and assess infection-free survival rate though Kaplan-Meier representation; p-value of Logrank test is indicated for each graph.

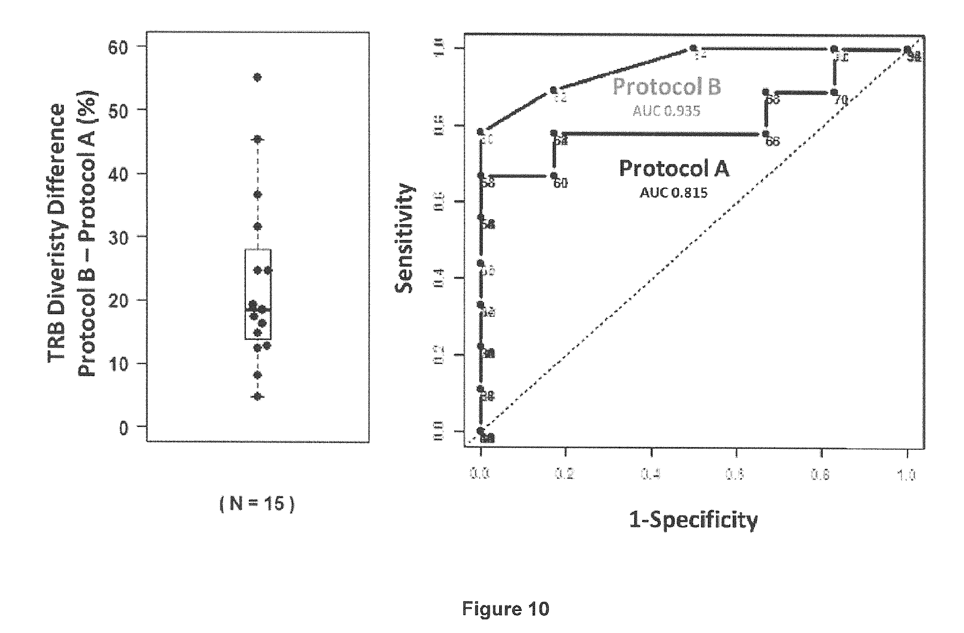

**Figure 10****:** TRB was evaluated twice through Protocol A and Protocol B for 15 DLBCL patients' samples. Left panel: Comparison between TRB diversity levels obtained using either Protocol A or Protocol B expressed by the difference of diversity (%). Right panel: for comparison of predictive performances between the 2 protocols, receiver operating characteristic (ROC) curve for inherent validity of TRB levels to discriminate infection risk was evaluated for each and the overlap is represented.

### EXAMPLES

### 1. Materials and Methods

### Combinatorial divervity analysis

Human TRB VJ and IGH VJ genes were designed according to the international ImMunoGeneTics (IMGT) database. Genomic DNA was extracted from human PBMC from patients affected by B-cell neoplasm such as NHL (DLBCL, FL) and chronic leukemia (CLL) after informed consent and concentrated using standard techniques. For each sample, multi-N-plex polymerase chain reaction (PCR) was performed using an upstream primer specific of all functional members of a given T-cell receptor β V (TRBV) family and a downstream primer specific of a given T-cell receptor β J (TRBJ) segment. For the same sample, an independent multi-N-plex was also applied for IGHV and IGHJ amplification. These assays allow for the simultaneous detection of several V-J rearrangements in the same reaction. Using this technique, it is possible to detect 276 different TRBV-TRBJ or 92 IGHV-IGHJ rearrangements. For TRB VJ combinatorial diversity assessment, PCR products were generated using iProof GC rich Master Mix (Bio-Rad) (Protocol A, specific technical protocol for RIPAL study) and by adding Tetramethylammonium chloride at a final concentration of 30mM (Protocol B, under definitive analytic validation for the use of different clinical applications). All V-J1, J2, and Jn products were separated as a function of their size. PCR products were separated on Lab on chip (HT DNA 12K LabChip Kit), by microfluidic migration (Labchip GX, PERKIN ELMER). Constel'ID® (ImmunID Technologies) software was used for further analytical studies including the generation of three-dimensional repertoire illustration. Results are expressed as percentages of detected rearrangements among the total 276 TRB and 92 IGH possible combinatorial rearrangements.

### Statistical analysis

Results are presented as box plots with individual values. Comparisons between groups were made using the nonparametric Mann- Whitney U test (Infection Yes vs. Infection No). The nonparametric Wilcoxon's paired test was used to assess variations between time points. Fisher's exact test or Yates'Chi2 test was used to compare proportions. Spearman's correlation test was used to assess correlations between TCR diversity values and clinical and biological variables. A receiver operating characteristic curve was performed to determine the cutoff values for TCR diversity and number of T lymphocytes with regard to prediction of infection by the end of M6. The best cutoff value was selected based on likelihood ratio and Youden's index. Using these thresholds, Kaplan-Meier survival curves were obtained, and differences in infection-free survival between groups were evaluated using log-rank test. Univariate analysis through the Cox model was used for the estimation of the hazard ratio and 95% CI. Statistical analyses were performed using the free statistical software package R. A p value < 0.05 was considered statistically significant.

### 2. RIPAL retrospective study result

RIPAL retrospective study is an observational biomedical research on Human ImmunTraCkeR® and Human Immun'IgH *in vitro* medical devices for Immunological Repertoire analysis in adult Patients affected with non-Hodgkin Lymphoma (NHL) or Chronic Lymphocytic Leukaemia (CLL). The retrospective analysis involved of all clinical and histologically confirmed NHL or CLL cases diagnosed during a few years in Hospices Civils de Lyon (HCL), and for which patient had a clearly signed Informed consent for the use of biological samples. In total of 86 included cases were analysed. In the analysis of infection occurrence and infection-free survival, the event was defined as the first febrile event without bacterial documentation or documented infection.

Table 1 below shows demographic, clinical and biological data from the analysed patients cohort and the 10 aged matched healthy volunteers used for immune repertoire comparisons. Main results of the analysis of this study are summarised in figure 1.

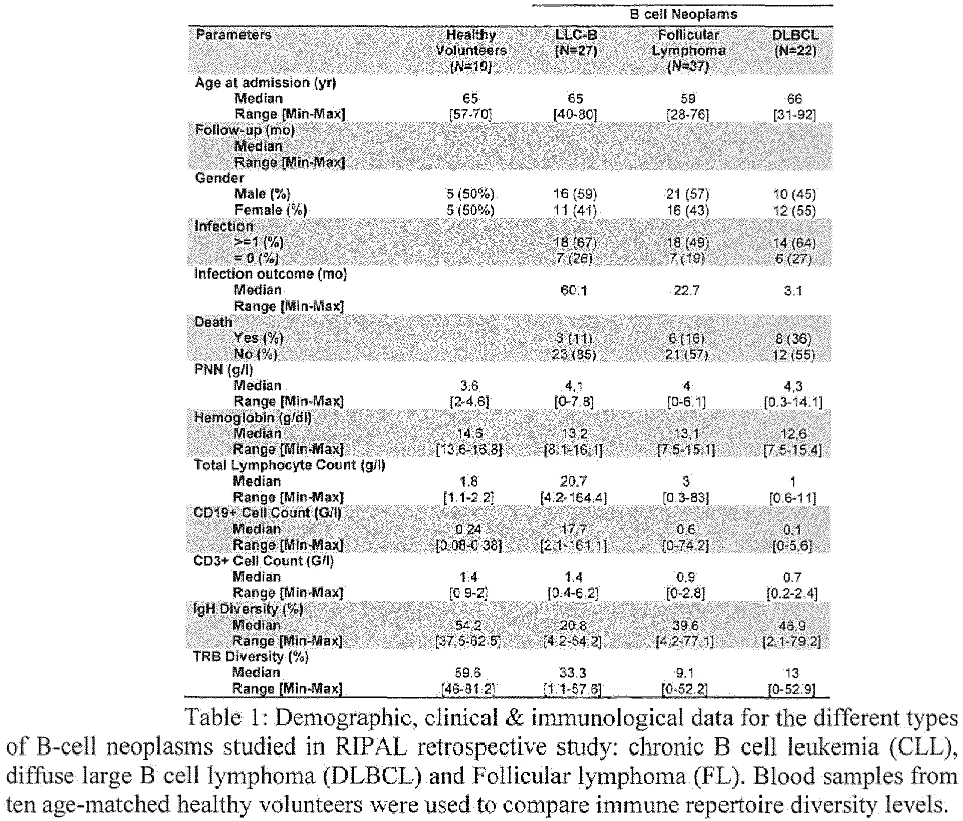

Both the IGH and TRB repertoires were found to be profoundly altered at diagnosis. All these values (except for IGH in DLBCL patients) were significantly different from those observed in age-matched healthy individuals (Wilcoxon test). Optimal thresholds of IGH and TRB diversity associated with the risk of infections were found using ROC curve analysis. In DLBCL patients (but not in FL and CLL patients), IGH diversity below 51% and TRB diversity below 10% were determined as optimal threshold for the occurrence of infections. When combining these 2 parameters (IGH <51% and TRB <10%), a highly significant difference in the risk of developing an infection during the 6 first months of treatment was observed (Log-rank test, p=.026). Of note, lymphopenia at diagnosis was not associated with this risk in this cohort.

These early results indicate that both the B- and T-cell receptors repertoires are profoundly modified in patients with lymphoid malignancies at diagnosis and that these modifications could be associated at least in DLBCL patients with the risk of developing an infection during treatment.

A prospective series to further expand and confirm these findings was done. The results presented below help to consider anti-infectious prophylaxis or treatment choice in patients with lymphoid malignancies. These analyses are also useful to investigate if the immune cells dysfunctions are associated with the clinical outcome of patients.

### 3. RIPAL prospective study results

RIPAL prospective study is an observational biomedical research on Human ImmunTraCkeR ® and Human Immun'IgH *in vitro* medical devices for Immunological Repertoire analysis in adult patients affected with non-Hodgkin Lymphoma (NHL) or Chronic Lymphocytic Leukaemia (CLL). Eligible patients had age of at least 18 years, had clinical and histologically confirmed NHL or CLL between 2009 and 2012. All patients were assigned for Rituximab-based chemotherapy; they were followed until death, loss to follow-up, or termination of the study for duration of 18 months. Blood samples were collected at diagnosis and subsequently after treatment at around day 21, corresponding to the 1st day of 2nd cycle (D1C2), and 3 (M3), 6 (M6) and 18 (M18) months after. In this study, the dynamics over time of the immune repertoires and other immune parameters evolution can be assessed (Fig. 2).

In the analysis of infection occurrence and infection-free survival, the event "infection" was defined as the first experienced febrile event diagnosed through fever, associated or not with bacterial, viral or fungal documentation, with or without obvious entrance door; no fever but obvious entrance door or clear documented infection. Subsequent infections for a same patient were not taken into account in this analysis.

The study included 87 consecutive patients with NHL or leukeamia that were monitored at diagnosis, during and after anti-CD20-based chemotherapy. Results exposed here were obtained from the analysis of 50 patients: 30 DLBCL and 20 FL. Several biological parameters were monitored including classic haematological and immunological parameters, T and B cell immune repertoires. Patients' clinical and biological description at diagnosis is depicted in Table 2.

Of note, according to the standard of care, G-CSF was administered concomitantly with Rituximab-based chemotherapy. Therefore, all patients have normal to low grade 1 neutropenia (between 1.5 to 2 G/L). In this context, grade 2 (between 1 to 1.5 G/L) or severe grade 3 (between 0.5 to 1 G/L) to 4 (<0.5 G/L) neutropenia, which has been associated with the occurrence of febrile events and some infections, are not observed in those patients.

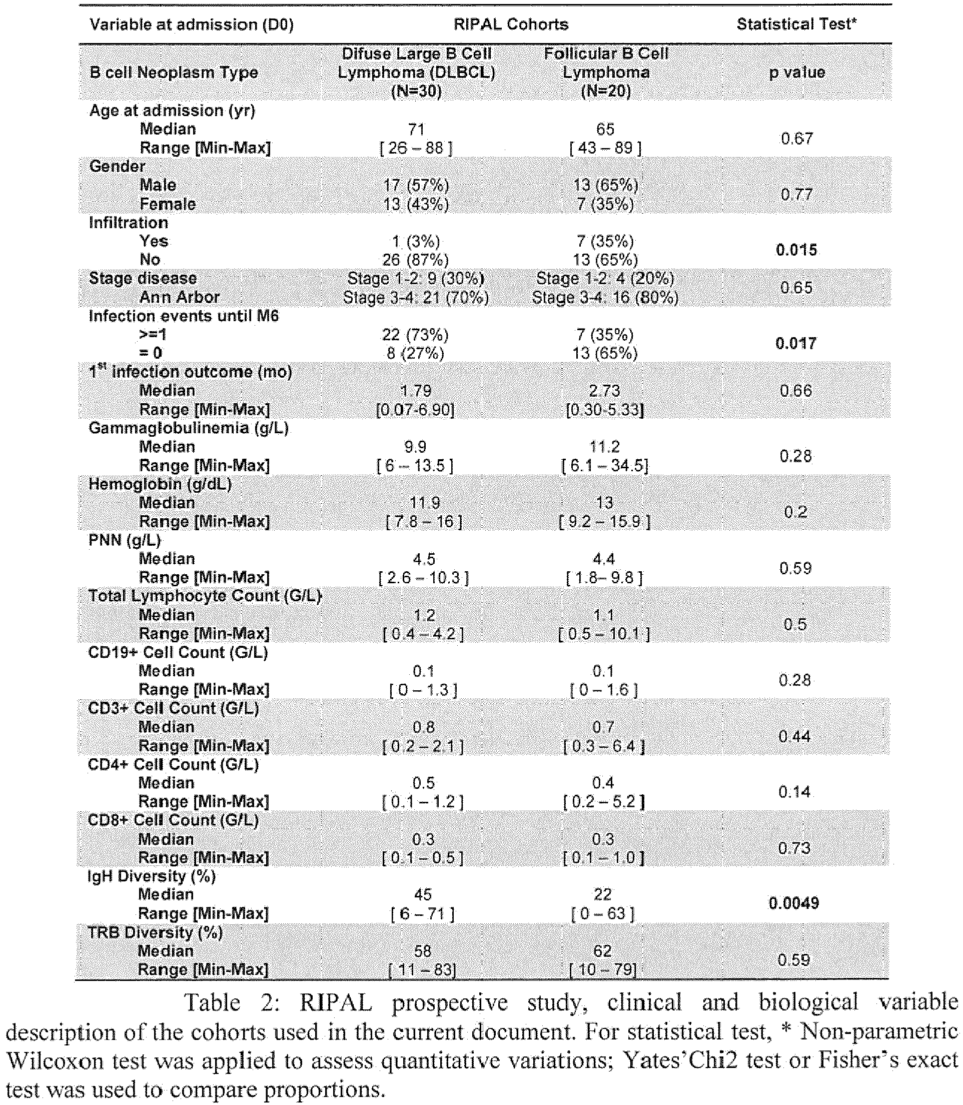

As depicted in Table 2, for most clinical and biological variables, no significant differences were found between DLBCL and FL patients at D0. However, a few significant differences were observed such as the proportion of infiltrated patients that is significantly higher in FL than DLBCL over the entire follow-up period; the number of 1st infection events is significantly higher in DLBCL than FL and finally, IGH diversity levels at D0 are significantly higher in DLBCL group than in FL patients.

In the next section, the occurrence of first infection was investigated and the analyses were first performed on DLBCL and FL cohorts separately, and then were done pooling both DLBCL and FL patients in order to increase the statistical power.

### 3.1. Diffuse Large B Cell Lymphoma (DLBCL) cohort

DLBCL patients were subdivided in 2 groups: those who developed at least one infection by the end of M6 and those who did not. For clinical variables such as Performance Status (PS), Ann Arbor stage, and B symptoms, the proportions of patients within the different classes were compared between the two groups and no significant differences could be observed. Quantitative differences were also tested between the 2 groups of patients, at D0 and at D1C2, for haemoglobin, total lymphocyte, PNN, CD19, CD3, CD4, CD8 counts and immune TRB and IGH repertoire diversity. Results of significant differences are shown in Table 3: only TRB diversity levels measured at D1C2 appear to be significantly lower in patients who experienced an infectious event by the end of M6.

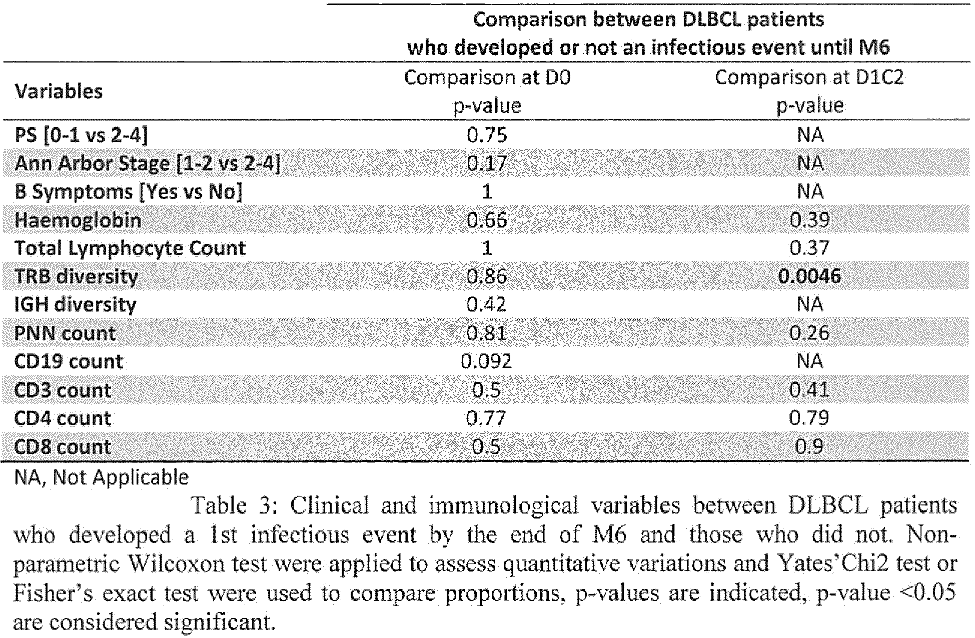

Despite no significant differences in IGH levels at D0 between DLBCL patients from RIPAL prospective study who developed or not infection, IGH diversity was tested for its capacity in discriminating between patients at higher risk of developing infection within 6 months. Results of this analysis indicate that a weak but significant capacity of discrimination is achieved in these conditions (Figure 3).

Of note, in the 2 studies the optimal threshold of IGH diversity that can stratify DLBCL patients at risk to develop an infection was defined by ROC analysis but was not the same for both studies. This is because samples from prospective and retrospective studies were analysed through different platforms and/or different protocols. Thus, the two studies cannot be pooled, and repertoire diversity levels should not be directly compared.

A novel aspect of RIPAL prospective study is the dynamic analysis of TRB and IGH repertoires over time. Indeed, for patients on RIPAL prospective study, diversity IGH and TRB levels were monitored during and after treatment at different time-points. This monitoring has highlighted an even more significant result obtained shortly after cycle 1 of treatment (DIC2; Table 4 and Figure 4A). Indeed, Receiver Operating Characteristic (ROC) analysis showed a strong discrimination performance of TRB diversity measured at D1C2, regarding the occurrence of infection (Figure 4B). Therefore, stratifying DLBCL patients according to the optimal Youden's index TRB threshold at 58%, showed that patients with TRB diversity >58% have less risk of infection occurrence by the end of M6 than the ones with TRB diversity <58% (Figure 4C). In addition, the median duration of infection-free survival was 1.45 months in the group TRB<58%, as compared with the group TRB >58% who didn't reach median duration over the follow-up period, corresponding to a significant (log rank p <0.001) hazard ratio (HR) for progression of 9.66 (95%CI 2.51-37.13) (Figure 4D).

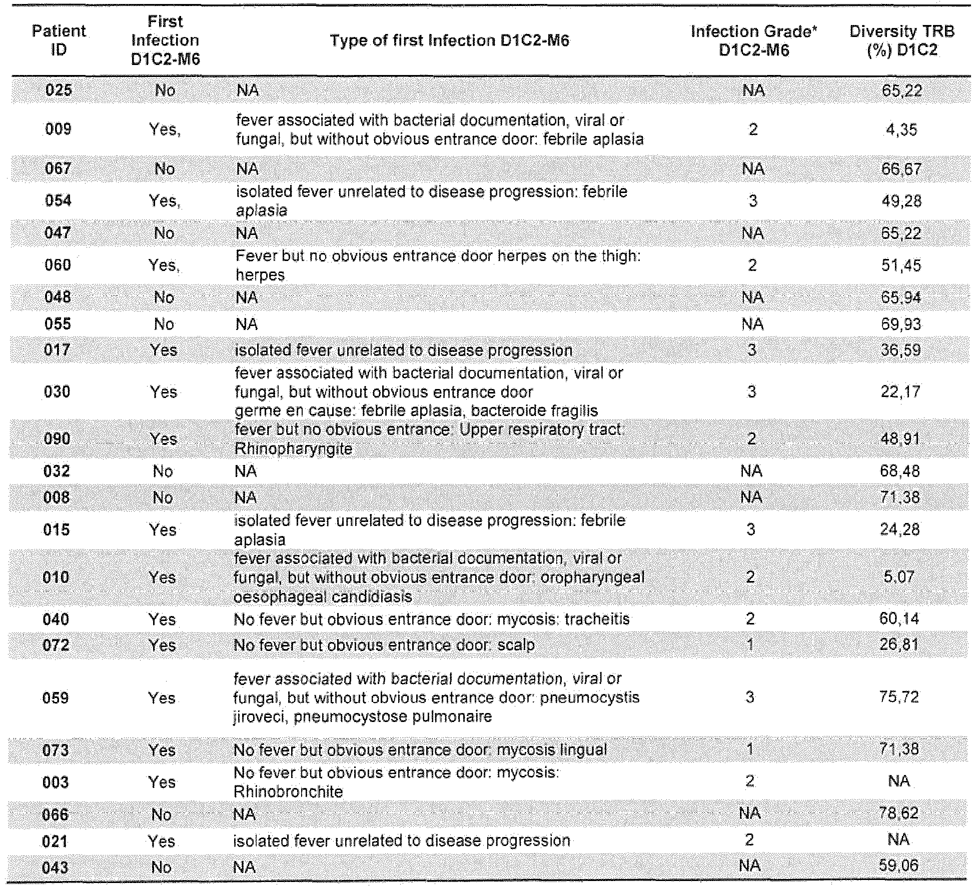

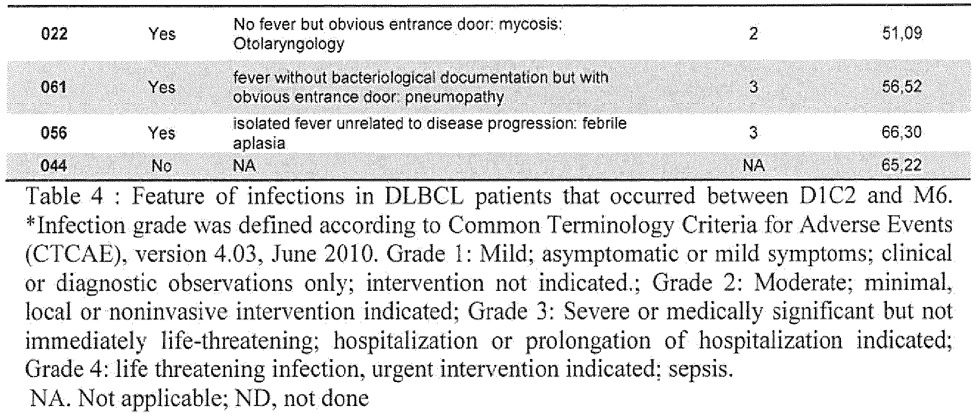

### 3.2. Follicular B Cell Lymphome (FL) Cohort

In order to assess if previous results obtained for DLBCL patients were applicable to other cohort of lymphoma, patients diagnosed with FL study and treated with Rituximab-based therapy, from RIPAL prospective, were analysed in the same conditions.

The same strategy of analysis was applied; therefore, FL patients were subdivided in 2 groups, those who developed at least one infection by the end of M6 and those who did not. Clinical variables (PS, Ann Arbor stage, and B symptoms) of patients with different classes were compared between the two groups and no significant differences could be observed. Quantitative differences were also tested between the 2 groups of patients, at D0 and at D1C2, for haemoglobin, total lymphocyte, PNN, CD19, CD3, CD4, CD8 counts and immune TRB and IGH repertoire diversity. Results of significant differences are highlighted in Table 5: only TRB diversity levels measured at D0 appear to be significantly lower in patients who experienced an infectious event by the end of M6 (Figure 5A). CD3+ CD4+ cell count appears to be barely close to significance (p-value ∼0.05).

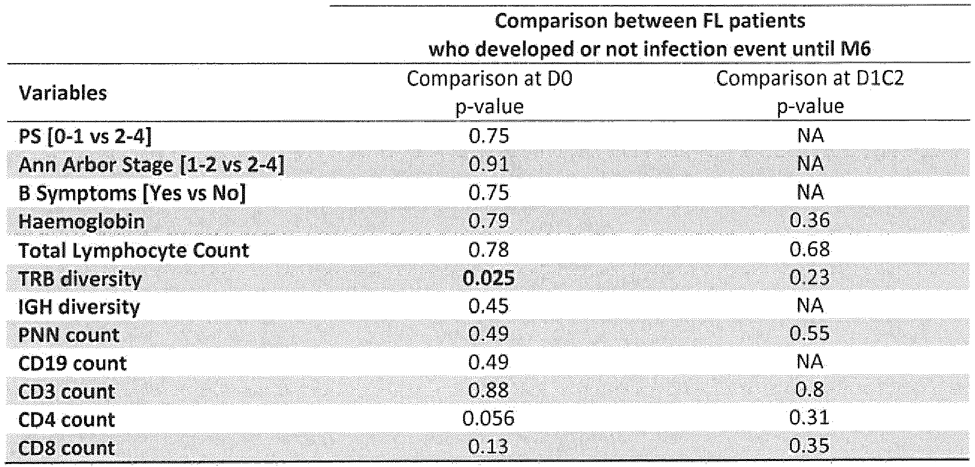

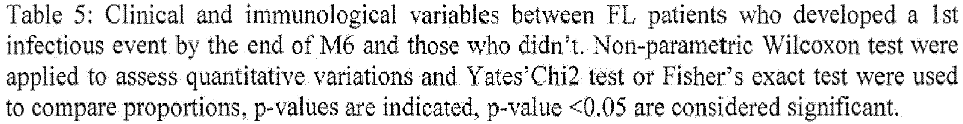

Despite the fact that no significant differences were observed for TRB diversity measured at D1C2 between infected and non-infected patients, predictivity of TRB levels at D1C2 to infection was still assessed. This analysis done in FL patients treated with Rituximab plus chemotherapy did not show significant results. However, a clear trend can be observed that is comparable to previous results obtained from DLBCL analysis (Figure 6), which seems to indicate that expected differences on TRB diversity at D1C2 were not achieved probably due to weak statistical power. Indeed, the group size is not sufficient to obtain a significant p-value (especially in the "Yes" group, n = 6). The power of the Wilcoxon test is estimated at 37% to highlight the difference in average of 15% between the two groups with these numbers.

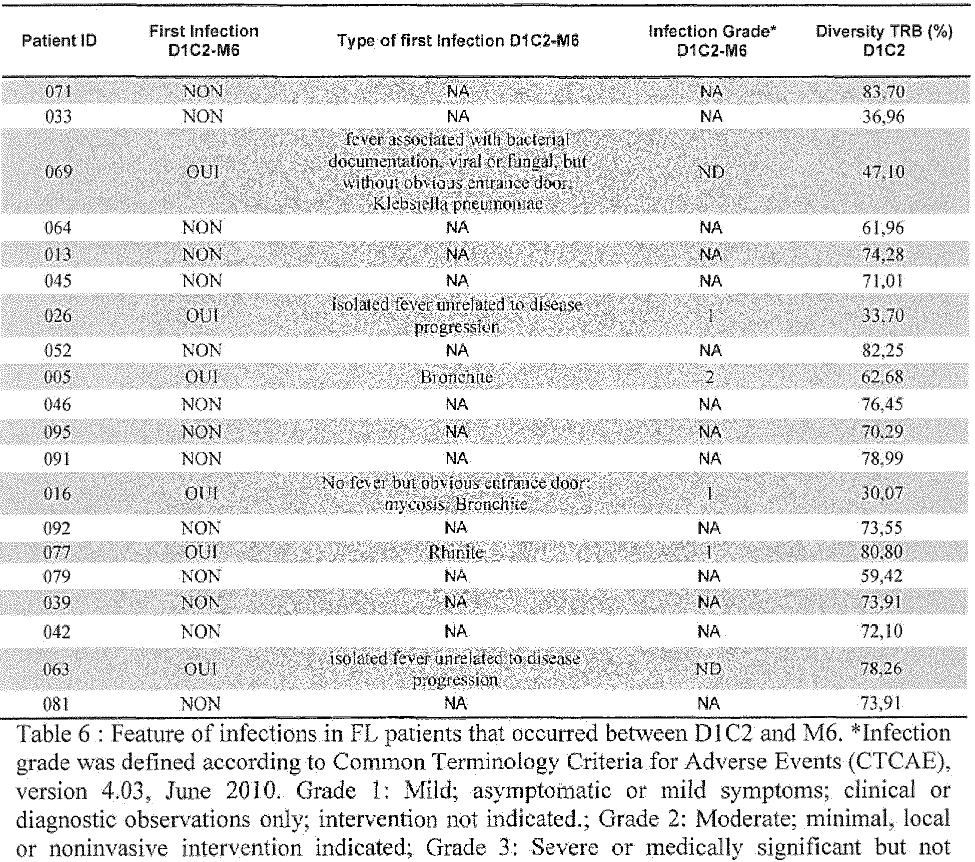

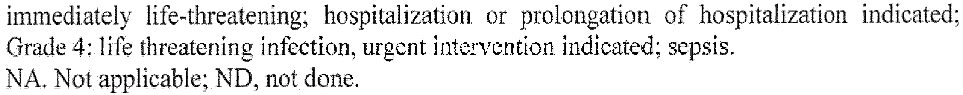

### 3.3. Pooled DLBC and FL cohorts

In order to increase statistical power, overall analyses were performed on pooled DLBCL plus FL patients for a total of 45 patients and 21 events. First, differences in clinical variables and biological parameters were investigated in pooled patients. For PS, Ann Arbor stage, and B symptoms, no differences were observed in the pooling group of patients compared to analysis done on individual DLBCL and FL cohorts. Quantitative differences were tested, at D0 and at D1C2, for haemoglobin, total lymphocyte, PNN, CD19, CD3, CD4, CD8 counts and immune TRB and IGH repertoire diversity. Results of significant differences are highlighted (in bold) in Table 7: TRB diversity levels measured at D0 (Figure 7) and at DIC2 (Figure 8) appear to be significantly lower in patients who experienced an infectious event by the end of M6. CD19+ cell count appears to be barely significant at D0.

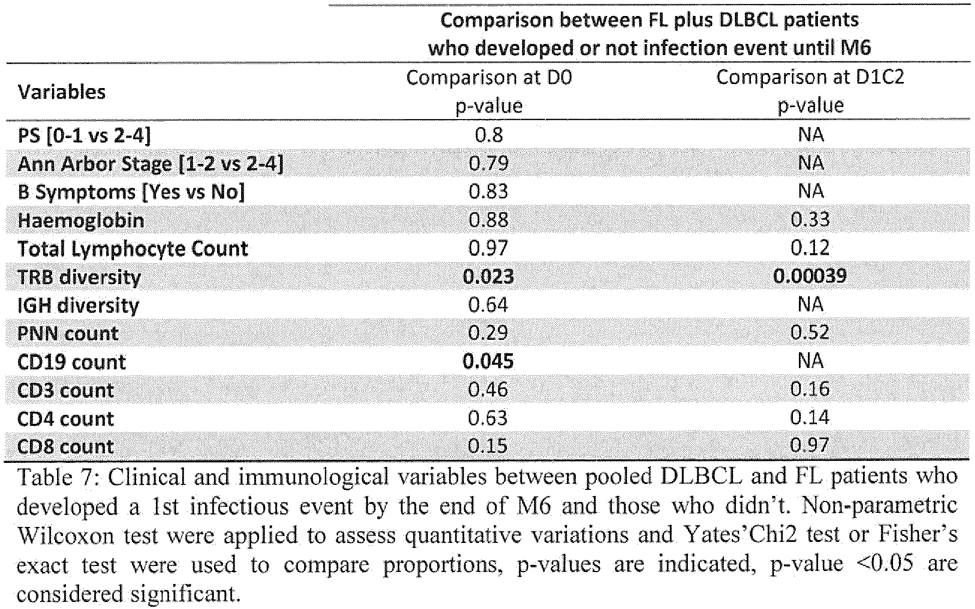

Analysis on TRB diversity at D0 done on pooled patients shows a significant difference between patients who developed at least one infection by the end of M6 and those who did not (Figure 7A). TRB diversity at D0 appears to be weakly discriminator for the occurrence of first infection until M6 (Figure 7B). ROC curve defined TRB at 56% as an optimal threshold and stratifying patients according to this cutoff, it is observed that the occurrence of first infection in the group of patients who have TRB levels ≥56% is later than in the group presenting TRB diversity <56%, but the difference is not significant (Figure 7C). However, in these conditions, a clear trend can still be observed.

Analysis on TRB diversity at D1C2 done on pooled patients shows a strong significant difference between patients who developed at least one infection by the end of M6 and those who did not (Figure 8A). The first results anticipate that TRB diversity is still discriminator for the occurrence of first infection until M6, and this is confirmed through ROC curve analysis (Figure 8B) that shows an AUC=0.811 and optimal Youden's index threshold at 58%. Stratifying patients according to this TRB threshold, it is observed that the occurrence of at least one infection in the group of patients who have TRB levels ≥58% is significantly lower than in the group of patients having a TRB diversity <58% (Figure 8C). In addition, Kaplan-Meier estimation based on this stratification shows that patients with TRB diversity lower than 58% have median duration of infection-free survival of 1.45 days as compared with the group TRB ≥58% who did not reach median duration over the follow-up period, corresponding to a strongly significant (log rank p <<0.05) hazard ratio (HR) for progression of 6.64 (95%CI 2.85-15.51).

### 3.4. Technical evolution of Human ImmunTracCkeRβ® test

So far, all results of RIPAL prospective study were obtained with a Human ImmunTraCkeRβ® test that uses a specific technical protocol, named Protocol A, which was analytically validated for the study. Nowadays, evaluation of T cell diversity through Human ImmunTraCkeRβ® kit has evolved to a more improved version, named Protocol B, which is under definitive analytic validation. This technical evolution due to improvement of technical process protocol, led to an increase of analytical sensitivity. Therefore, part of the presented results was assessed using the new version (Protocol B) of the Human ImmunTraCkeRβ® test. To do so, 15 D21 samples from DLBCL patients were randomly selected to be reanalysed using Protocol B and results were compared between the two protocols.

As shown in Figure 9, despite changes in the levels of TRB diversity obtained using either protocol A or protocol B, patients who experienced an infectious event by the end of M6 still have TRB diversity levels significantly lower than those who did not (Figure 9, left panels). Using receiver operating characteristic (ROC) curves, discrimination capacity previously observed for protocol A is maintained when Protocol B is used (Figure 9, middle panels). Youden index indicates that optimal thresholds are different according to the type of protocol used, related to differences in analytical performances between the two protocols. Therefore, improvements done in Protocol B have led to a different optimal cut-off point of discrimination; using Youden index, the optimal threshold of 80% is obtained for Protocol B. Comparing Infection-free survival for each protocol, using corresponding optimal threshold, shows a significant difference in the survival rate of Kaplan-Meier representation in both cases (Figure 9, right panels).

TRB diversity levels were compared for the 15 samples between protocol A and B. To do so, for each sample the difference of diversity was calculated through the difference of diversity levels obtained through protocol B minus protocol A, as indicated in Figure 10 (left panel). For all the samples tested, the measured diversity was higher using protocol B than A; the median increase is 19%, going from minimum difference of 5% to a maximum of 55% (Figure 10 left panel). Overlapping ROC curves, it can be observed that TRB diversity levels obtained through Protocol B have better discrimination capacity than previous Protocol A, as assessed by the Area under the ROC curve (AUC), which is considered as an effective measure of inherent validity of a diagnostic test: AUC from ROC curve obtained from protocol B is higher than AUC from protocol B (Figure 10, right panels). These analyses illustrate how technical changes and improvements can affect analytical performances without affecting clinical validity of the biomarker.

### REFERENCES

Avivi, I., Stroopinsky, D., and Katz, T. (2013), Anti-CD20 monoclonal antibodies: Beyond B-cells. Blood Rev. 27, 217-223.
Dal-Bo, M., Del Giudice, I., Bomben, R., Capello, D., Bertoni, F., Forconi, F., Laurenti, L., Rossi, D., Zucchetto, A., Pozzato, G., et al. (2011). B-cell receptor, clinical course and prognosis in chronic lymphocytic leukaemia: the growing saga of the IGHV3 subgroup gene usage. Br. J. Haematol. 153, 3-14.
Burger, J.A., Ghia, P., Rosenwald, A., and Caligaris-Cappio, F. (2009). The microenvironment in mature B-cell malignancies: a target for new treatment strategies. Blood 114, 3367-3375.
Cabanillas, F., Liboy, I., Pavia, O., and Rivera, E. (2006). High incidence of non-neutropenic infections induced by rituximab plus fludarabine and associated with hypogammaglobulinemia: a frequently unrecognized and easily treatable complication. Ann. Oncol. Off. J. Eur. Soc. Med. Oncol. ESMO 17, 1424-1427.
Cullen, M., Steven, N., Billingham, L., Gaunt, C., Hastings, M., Simmonds, P., Stuart, N., Rea, D., Bower, M., Fernando, I., et al. (2005). Antibacterial Prophylaxis after Chemotherapy for Solid Tumors and Lymphomas. N. Engl. J. Med. 353, 988-998.
Cullen, M.H., Billingham, L.J., Gaunt, C.H., and Steven, N.M. (2007). Rational Selection of Patients for Antibacterial Prophylaxis After Chemotherapy. J. Clin. Oncol. 25, 4821-4828.
Gascoyne, R.D. (2014). The biology of diffuse large B cell lymphoma (dlbcl). Pathology (Phila.) 46 Suppl 1, S32.
Ghielmini, M., Vitolo, U., Kimby, E., Montoto, S., Walewski, J., Pfreundschuh, M., Federico, M., Hoskin, P., McNamara, C., Caligaris-Cappio, F., et al. (2013). ESMO Guidelines consensus conference on malignant lymphoma 2011 part 1: diffuse large B-cell lymphoma (DLBCL), follicular lymphoma (FL) and chronic lymphocytic leukemia (CLL). Ann. Oncol. Off. J. Eur. Soc. Med. Oncol. ESMO 24, 561-576.
Hallek, M. (2013). Chronic lymphocytic leukemia: 2013 update on diagnosis, risk stratification and treatment. Am. J. Hematol. 88, 803-816.
Al-Katib, A., Raufi, and Ebrahim (2013). Targeting CD19 in B-cell lymphoma: emerging role of SAR3419. Cancer Manag. Res. 225.
Malek, S. (2013). Molecular biomarkers in chronic lymphocytic leukemia. Adv. Exp. Med. Biol. 792, 193-214.
Morrison, V.A. (2010). Infectious complications of chronic lymphocytic leukaemia: pathogenesis, spectrum of infection, preventive approaches. Best Pract. Res. Clin. Haematol. 23, 145-153.
Morton, L.M., Whang, S.S., Devesa, S.S., Hartge, P., Weisenburger, D.D., and Linet, M.S. (2006). Lymphoma incidence patterns by WHO subtype in the United States, 1992-2001. Blood 107, 265-276.
Neumann, S., Krause, S.W., Maschmeyer, G., Schiel, X., von Lilienfeld-Toal, M., Infectious Diseases Working Party (AGIHO), and German Society of Hematology and Oncology (DGHO) (2013). Primary prophylaxis of bacterial infections and Pneumocystis jirovecii pneumonia in patients with hematological malignancies and solid tumors: guidelines of the Infectious Diseases Working Party (AGIHO) of the German Society of Hematology and Oncology (DGHO). Ann. Hematol. 92, 433-442.
Récher, C., Coiffier, B., Haioun, C., Molina, T.J., Fermé, C., Casasnovas, O., Thiéblemont, C., Bosly, A., Laurent, G., Morschhauser, F., et al. (2011). Intensified chemotherapy with ACVBP plus rituximab versus standard CHOP plus rituximab for the treatment of diffuse large B-cell lymphoma (LNH03-2B): an open-label randomised phase 3 trial. The Lancet 378, 1858-1867.
Roschewski, M., Dunleavy, K., and Wilson, W.H. (2014). Moving Beyond R-CHOP for Diffuse Large B-cell Lymphoma. Leuk. Lymphoma.
Salles, G., and Ghesquières, H. (2012). Current and future management of follicular lymphoma. Int. J. Hematol. 96, 544-551.
Siegel, R., Naishadham, D., and Jemal, A. (2013). Cancer statistics, 2013. CA. Cancer J. Clin. 63, 11-30.
Tadmor, T., Bari, A., Sacchi, S., Marcheselli, L., Liardo, E.V., Avivi, I., Benyamini, N., Attias, D., Pozzi, S., Cox, M.C., et al. (2013). Monocyte count at diagnosis is a prognostic parameter in diffuse large B-cell lymphoma: a large multicenter study involving 1191 patients, in the pre and post rituximab era. Haematologica.
Trecarichi, E.M., and Tumbarello, M. (2014). Antimicrobial-resistant Gram-negative bacteria in febrile neutropenic patients with cancer: current epidemiology and clinical impact. Curr. Opin. Infect. Dis. 27, 200-210.

## Claims

1. An *in vitro* method of predicting the occurrence of a febrile and/or infectious event in a patient suffering from a B-cell neoplasm, comprising: measuring diversity of the immune repertoire in a biological sample from said patient and comparing the measured diversity to a predetermined threshold, wherein a measured diversity lower than said threshold is indicative of an increased risk of occurrence of a febrile and/or infectious event, and a measured diversity higher than said threshold is indicative of a decreased risk of occurrence of a febrile and/or infectious event.

2. An *in vitro* method of determining the risk-benefit balance of an anti-CD20-based chemotherapy for a patient suffering from a B-cell neoplasm, comprising a step of performing the method of claim 1, wherein the potential benefits of the anti-CD20-based chemotherapy outweigh its risks for the patient if said patient has a decreased risk of occurrence of a febrile and/or infectious event.

3. The method of claim 1 or claim 2, wherein a measured diversity lower than said predetermined threshold indicates that the patient needs an antibacterial and/or antifungal prophylactic treatment or another preventive treatment, and a measured diversity higher than said threshold indicates that the patient does not need such a prophylactic treatment or other preventive treatment.

4. The method of any of claims 1 to 3, wherein said anti-CD20-based chemotherapy comprises administrating an anti-CD-20 monoclonal antibody.

5. The method of any of claims 1 to 4, wherein said anti-CD20-based chemotherapy is R-CHOP or R-ACVBP.

6. The method according to any of the preceding claims, wherein the biological sample has been collected between 18 and 24 days after the first administration of said treatment.

7. The method according to any of the preceding claims, wherein said cancer patient suffers from a B lymphoma.

8. The method according to claim 7, wherein said cancer patient suffers from a large B cell lymphoma (DLBCL) or a follicular lymphoma (FL).

9. The method according to any of the preceding claims, wherein said biological sample is selected from the group consisting of a whole blood sample, a blood clot, PBMCs and a tissue biopsy.

10. The method according to any of the preceding claims, wherein said measured diversity is TCR diversity.

11. The method according to any of the preceding claims, wherein said measured TCR diversity comprises combinatorial TRB diversity.

12. The method according to any of the preceding claims, wherein said measured diversity of the immune repertoire is measured by a multiplex TCR assay allowing the simultaneous detection of at least 20 TRBV-TRBJ rearrangements.

13. The method according to any of the preceding claims, wherein a patient suffering from a B lymphoma and having a combinatorial TRB diversity ≤ 58% 18 to 24 days after the first administration of an anti-CD20-based chemotherapy is identified at risk of infection and as being in need of an antibacterial and/or antifungal prophylactic treatment healthy life style recommendations or other preventive treatments.

14. The method according to any of the preceding claims, wherein a patient suffering from DLBCL and having a combinatorial TRB diversity ≤ 58% 18 to 24 days after the first administration of an anti-CD20-based chemotherapy is identified as being in need of an antibacterial and/or antifungal prophylactic treatment healthy life style recommendations or other preventive treatments.

15. The method according to any of the preceding claims, wherein a patient suffering from FL and having a combinatorial TRB diversity ≤ 64% 18 to 24 days after the first administration of a treatment comprising an anti-CD20 molecule is identified as being in need of an antibacterial and/or antifungal prophylactic treatment, healthy life style recommendations or other preventive treatments.
